# EUROPEAN PATENT APPLICATION

(11) **EP 2 685 260 A1**
(43) Date of publication of application: **15.01.2014**
(21) Application number: 12175572.2
(22) Date of filing: 09.07.2012
(51) Int. Cl.: G01N 33/50, G01N 33/58, A61K 49/00

(54) **Direct and quantitative detection of targets in living cells**

(71) Applicant: Ludwig-Maximilians-Universität München, 80539 München (DE)
(72) Inventor: Leonhardt, Heinrich, 80469 München (DE); Helma, Jonas, 81667 München (DE); Huber, Andreas, 80339 München (DE)
(74) Representative: Vossius & Partner

(57) **Abstract**

The present invention relates to a method for the detection of the presence or absence of a target in a cell, the method comprising the steps of: (a) introducing into a cell (i) a first molecule comprising or consisting of a first single-domain antibody binding to a first portion of the target to be detected and a first detection moiety or a nucleic acid encoding said first molecule in expressible form; and (ii) a second molecule comprising or consisting of a second single-domain antibody binding to a second portion of said target to be detected and a second detection moiety or a nucleic acid encoding said second molecule in expressible form, wherein said first portion and said second portion are spatially distinct to allow simultaneous binding of said first and said second single-domain antibody to said target; and wherein said first and said second detection moiety are in combination suitable for generating a detectable fluorescent signal occurring as a consequence of fluorescent moiety Förster resonance energy transfer (FRET) or bimolecular fluorescence complementation (BiFC) when in spatial proximity of each other upon binding of said first molecule to said first portion and said second molecule to said second portion of the target to be detected; (b) subjecting the cell of step (a) to conditions suitable for generating said detectable fluorescent signal; and (c) analysing the cell of step (b) for the presence of said detectable fluorescent signal generated by way of fluorescent moiety Förster resonance energy transfer (FRET) or bimolecular fluorescence complementation (BiFC) occurring between said first and said second detection moiety, and/or for a decrease in fluorescence lifetime of the detection moiety acting as energy donor as a consequence of Förster resonance energy transfer (FRET) occurring between said first and said second moiety in comparison to a control, wherein if said detectable fluorescent signal and/or said decrease in fluorescence lifetime can be detected, it is indicative for the presence of said target to be detected in said cell, and wherein if said detectable fluorescent signal and/or decrease in fluorescence lifetime cannot be detected in said cell, it is indicative for the absence of said target to be detected in said cell. Further, the invention relates to a composition comprising said first or second molecule or one or more nucleic acids encoding said first or second molecule or a kit comprising the latter composition, molecules or nucleic acids and, optionally, instructions for use.

## Description

The present invention relates to a method for the detection of the presence or absence of a target in a cell, the method comprising the steps of: (a) introducing into a cell (i) a first molecule comprising or consisting of a first single-domain antibody binding to a first portion of the target to be detected and a first detection moiety or a nucleic acid encoding said first molecule in expressible form; and (ii) a second molecule comprising or consisting of a second single-domain antibody binding to a second portion of said target to be detected and a second detection moiety or a nucleic acid encoding said second molecule in expressible form, wherein said first portion and said second portion are spatially distinct to allow simultaneous binding of said first and said second single-domain antibody to said target; and wherein said first and said second detection moiety are in combination suitable for generating a detectable fluorescent signal occurring as a consequence of fluorescent moiety Förster resonance energy transfer (FRET) or bimolecular fluorescence complementation (BiFC) when in spatial proximity of each other upon binding of said first molecule to said first portion and said second molecule to said second portion of the target to be detected; (b) subjecting the cell of step (a) to conditions suitable for generating said detectable fluorescent signal; and (c) analysing the cell of step (b) for the presence of said detectable fluorescent signal generated by way of fluorescent moiety Förster resonance energy transfer (FRET) or bimolecular fluorescence complementation (BiFC) occurring between said first and said second detection moiety, and/or for a decrease in fluorescence lifetime of the detection moiety acting as energy donor as a consequence of Förster resonance energy transfer (FRET) occurring between said first and said second moiety in comparison to a control, wherein if said detectable fluorescent signal and/or said decrease in fluorescence lifetime can be detected, it is indicative for the presence of said target to be detected in said cell, and wherein if said detectable fluorescent signal and/or decrease in fluorescence lifetime cannot be detected in said cell, it is indicative for the absence of said target to be detected in said cell. Further, the invention relates to a composition comprising said first or second molecule or one or more nucleic acids encoding said first or second molecule or a kit comprising the latter composition, molecules or nucleic acids and, optionally, instructions for use.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Current methods for quantification of cellular protein levels rely on antibody-based detection techniques such as Western Blot analysis and Enzyme-Linked-Immunosorbent Assay (ELISA). Quantitative diagnostic tests (e.g. PSA tests and HIV p24 tests) are often based on antibody sandwich assays, where an immobilized monoclonal antibody detects a specific epitope within the target and a second reporter antibody detects a different epitope of the target and thereby verifies its presence. However, these methods are restricted to *in vitro* applications. In live cells, Fluorescence Resonance Energy Transfer (FRET) and Bimolecular Fluorescent Complementation (BiFC) are powerful tools to measure and quantify protein interactions. FRET and BiFC rely on two reporter components residing in close spatial proximity to specifically allow nonradiative excitation of a fluorescent acceptor by a fluorescent donor or functional complementation of split fluorescent proteins (FPs), respectively. However, in live cells both methods require the recombinant tagging of a given target with FPs and cannot be applied to specific endogenous proteins. While nanobodies (single-domain antibodies) coupled to fluorescent proteins are used to detect cellular proteins, the visual read-out necessarily relies on the detection reagent itself, thus endogenous structures are only visualized indirectly. As a consequence, only qualitative results regarding localization and compartmental allocation but no quantitative results can simultaneously be obtained with said method.

The technical problem underlying the present invention was to identify alternative and/or improved means and methods for detection and optional quantification of targets within living cells.

The solution to this technical problem is achieved by providing the embodiments characterized in the claims.

Accordingly, the present invention relates in a first embodiment to a method for the detection of the presence or absence of a target in a cell, the method comprising the steps of: (a) introducing into a cell (i) a first molecule comprising or consisting of a first single-domain antibody binding to a first portion of the target to be detected and a first detection moiety or a nucleic acid encoding said first molecule in expressible form; and (ii) a second molecule comprising or consisting of a second single-domain antibody binding to a second portion of said target to be detected and a second detection moiety or a nucleic acid encoding said second molecule in expressible form, wherein said first portion and said second portion are spatially distinct to allow simultaneous binding of said first and said second single-domain antibody to said target; and wherein said first and said second detection moiety are in combination suitable for generating a detectable fluorescent signal occurring as a consequence of fluorescent moiety Förster resonance energy transfer (FRET) or bimolecular fluorescence complementation (BiFC) when in spatial proximity of each other upon binding of said first molecule to said first portion and said second molecule to said second portion of the target to be detected; (b) subjecting the cell of step (a) to conditions suitable for generating said detectable fluorescent signal; and (c) analysing the cell of step (b) for the presence of said detectable fluorescent signal generated by way of fluorescent moiety Förster resonance energy transfer (FRET) or bimolecular fluorescence complementation (BiFC) occurring between said first and said second detection moiety, and/or for a decrease in fluorescence lifetime of the detection moiety acting as energy donor as a consequence of Förster resonance energy transfer (FRET) occurring between said first and said second moiety in comparison to a control, wherein if said detectable fluorescent signal and/or said decrease in fluorescence lifetime can be detected, it is indicative for the presence of said target to be detected in said cell, and wherein if said detectable fluorescent signal and/or decrease in fluorescence lifetime cannot be detected in said cell, it is indicative for the absence of said target to be detected in said cell.

The term "target" in accordance with the method of the invention can be any entity within a cell that is accessible for and can be bound by single-domain antibodies. The target may be a target occurring endogenously within the cell that may or may not be artificially modified and/or be introduced into the cell by natural (e.g., infection, phagocytosis, etc.) or artificial means (e.g., chemical transduction, etc.). As such, a target in accordance with the invention can relate to any organic and/or inorganic entity. An organic target can be, e.g., a proteinaceous target, a peptide, a nucleic acid molecule, a hormone, a lipid or any combination thereof, but may not necessarily be a molecule, while inorganic targets can, for example, be metals, inorganic salts or any combination thereof. In line with the foregoing, a complex resulting from the interaction of binding partners ― which may belong to any of the above mentioned entities - is considered a target in accordance with the invention. Further, and when not relating to a molecule, the term "target" also includes a certain conformation, a posttranslational modification or a state of activation (e.g., phosphorylation state, methylation state, acetylation state, ubiquitination state) of any of the above mentioned entities such as, e.g., of a protein.

A "cell" in accordance with the invention can be any cell. It is understood by the person skilled in the art that depending on the goal to be achieved with the method described, different cells may be more suitable than others. The cell may be an archaea cell, a prokaryotic cell or a eukaryotic cell such as a plant, an animal or a fungus cell. For example, the cell is a mammalian cell. The term "mammalian cell" as used herein, is well known in the art and refers to any cell belonging to an animal that is grouped into the class of mammalia. To the extent human cells are envisaged for use in the method of the invention, it is preferred that such human cell is not obtained from a human embryo, in particular not via methods entailing destruction of a human embryo. On the other hand, human embryonic stem cells are at the skilled person's disposal such as taken from existent embryonic stem cell lines commercially available. Accordingly, the present invention may be worked with human embryonic stem cells without any need to use or destroy a human embryo. Alternatively, or instead of human embryonic stem cells, pluripotent cells that resemble embryonic stem cells such induced pluripotent stem (iPS) cells may be used, the generation of which is state of the art (Hargus et al., 2010; Jaenisch, 2008; Saha, 2009). The term "cell" as used herein can refer to a single and/or isolated cell or to a cell that is part of a multicellular entity such as a tissue, an organism or a cell culture. In other words the method can be performed *in vivo, ex vivo* or *in vitro.* Depending on the particular goal to be achieved with the method of the invention, cells of different mammalian subclasses such as prototheria or theria may be used. For example, within the subclass of theria, preferably cells of animals of the infraclass eutheria, more preferably of the order primates, artiodactyla, perissodactyla, rodentia and lagomorpha are used in the method of the invention. Furthermore, within a species one may choose a cell to be used in the method of the invention based on the tissue type and/or capacity to differentiate, this equally depending on the goal to be achieved by altering the genome. Three basic categories of cells make up the mammalian body: germ cells, somatic cells and stem cells. A germ cell is a cell that gives rise to gametes and thus is continuous through the generations. Stem cells can divide and differentiate into diverse specialized cell types as well as self renew to produce more stem cells. In mammals there are two main types of stem cells: embryonic stem cells and adult stem cells. Somatic cells include all cells that are not a gametes, gametocytes or undifferentiated stem cells. The cells of a mammal can also be grouped by their ability to differentiate. A totipotent (also known as omnipotent) cell is a cell that is able to differentiate into all cell types of an adult organism including placental tissue such as a zygote (fertilized oocyte) and subsequent blastomeres, whereas pluripotent cells, such as embryonic stem cells, cannot contribute to extraembryonic tissue such as the placenta, but have the potential to differentiate into any of the three germ layers endoderm, mesoderm and ectoderm. Multipotent progenitor cells have the potential to give rise to cells from multiple, but limited number of cell lineages. Further, there are oligopotent cells that can develop into only a few cell types and unipotent cells (also sometimes termed a precursor cell) that can develop into only one cell type. There are four basic types of tissues: muscle tissue, nervous tissue, connective tissue and epithelial tissue that a cell to be used in the method of the invention can be derived from, such as for example hematopoietic stem cells or neuronal stem cells. Any of the above cells may in accordance with the invention be diseased and non-diseased cells or cells that have been artificially (e.g., genetically) modified.

Exemplary cell types that may be used in accordance with the invention are lung cells, dermal cells, blood cells, lymphatic cells, neuron cells, kidney cells, liver cells, muscle cells, gland cells, sensory cells, cancer cells, embryonic and adult stem cells, as well as induced pluripotent stem (iPS) cells.

Cells to be used may originate from established cell lines but may also include cell of a primary cell line established from a tissue sample. Preferably, the cells originate from the same cell line or are established from the same tissue. Methods to obtain samples from various tissues and methods to establish primary cell lines are well-known in the art (Jones GE, Wise CJ., "Establishment, maintenance, and cloning of human dermal fibroblasts." Methods Mol Biol. 1997;75:13-21). Suitable cell lines may also be purchased from a number of suppliers such as, for example, the American tissue culture collection (ATCC), the German Collection of Microorganisms and Cell Cultures (DSMZ) or PromoCell GmbH, Sickingenstr. 63/65, D-69126 Heidelberg.

The feature "introducing into a cell" as used in the context of the method of the invention is meant to refer to the step of supplying the cell with the first and second molecule or one or more nucleic acid molecules encoding the latter so that these molecules are located inside the cell as a result of said step. Said introduction can be effected with a variety of methods, wherein the suitability of a certain method depends, for example, on the nature of the material to be introduced (e.g., protein or nucleic acid) or the cell that is used. The person skilled in the art is in the position to optimize existing methods for introducing proteins and/or nucleic acids by using routine measures. For example, and in the case of nucleic acid molecules, nucleic acid molecules may be designed for direct introduction or for introduction via lipid-based transfection reagents, calcium-phosphate precipitation, liposomes, phage vectors or viral vectors (e.g. adenoviral, retroviral) into the cell. Preferably, introduction is achieved via a lipid-based transfection method. In the case that the first and/or second molecule is to be introduced into a cell in the form of a protein or partial protein, in case the detection moiety is not a protein, several methods such as, e.g., permeabilization using Sendai virus, trypsination, osmotic shock, microinjection or electroporation can be employed. Moreover and similar to nucleic acids, proteins may be delivered into cells via lipid-based transfection/proteofection reagents. Alternatively, the first and/or second molecule carries a genetically encoded membrane translocation signal such as a cell-penetrating peptide (CPP). For example, the HIV-1 Tat peptide or the antennapedia peptide penetratin enable such cellular uptake (Deshayes et al., 2005). The advantage of a corresponding strategy is that it does not require any additional treatment. Preferably, the method allows introduction of both the first and second molecule into more than one cell simultaneously such as achievable, for example, by electroporation, lipid-based transfection or by membrane translocation signals.

The "first" and "second molecule" (also referred to herein as first and second molecule pair) are in their minimal setup structurally the same in that both comprise at least or consist of a single-domain antibody and a detection moiety. Depending on the specific goal to be achieved with the method of the invention, the skilled person may add further moieties to the first and/or the second molecule. The first and second molecule may independently from each other take the form of fusion proteins in case the first and second detection moieties are made up of fluorescent proteins (in the case FRET is to occur) or complementing fragments of one fluorescent protein (in the case BiFC is to occur) and can be prepared, e.g., by fusing a first nucleic acid encoding a given single-domain antibody in frame to a second nucleic acid molecule, encoding a detection moiety, wherein the first nucleic acid molecule is located 5' of the second nucleic acid molecule and wherein the nucleic acid molecules are optionally separated by a further nucleic acid molecule encoding a linker of, e.g., at least one amino acid residue; and expressing the fused nucleic acid molecule encoding said first or second molecule as fusion protein in a cell or cell free extract. The term "linker" as used in accordance with the present invention in the context of DNA sequences encoding a fusion protein relates to a sequel of amino acids (i.e. peptide linkers), which separate the single-domain antibody from the detection moiety. Peptide linkers as envisaged by the present invention are (poly)peptide linkers of at least 1 amino acid in length. Preferably, the linkers are 1 to 100 amino acids in length. More preferably, the linkers are 5 to 50 amino acids in length and even more preferably, the linkers are 10 to 20 amino acids in length. The nature, i.e. the length and/or amino acid sequence of the linker may modify or enhance the stability and/or solubility of the fusion protein. The length and sequence of a linker depends on the composition of the respective fusion protein with respect to the single-domain antibody and the detection moiety making up a molecule of the invention. Testing the suitability of different linkers can be achieved in routine experiments. For example, the properties of a molecule of the invention can easily be tested by comparing the binding affinity of the single-domain antibodies and/or the fluorescent properties of the molecule of the invention, e.g., with or without a linker or vis-à-vis different linkers. Preferably, the linker is a flexible linker using e.g. the amino acids alanine and serine or glycine and serine. Preferably the linker sequences are (Gly₄Ser)₄, or (Gly₄Ser)₃. The expression system may be prokaryotic or eukaryotic. A typical mammalian expression vector contains the promoter element, which mediates the initiation of transcription of mRNA, the protein coding sequence, and signals required for the termination of transcription and polyadenylation of the transcript. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109)), pEGFP-C1, pEGFP-N1, pEYFP-c1, pEYFP-N1 (Takara Bio, Kyoto, Japan). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the recombinant (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al., 1991; Bebbington et al., 1992). Using these markers, the mammalian cells are grown in selective medium and the cells with the highest resistance are selected. The expression vectors will preferably include at least one selectable marker. Such markers include dihydrofolate reductase, G418 or neomycin resistance for eukaryotic cell culture and tetracycline, kanamycin or ampicillin resistance genes for culturing in *E*. *coli* and other bacteria. Representative examples of appropriate hosts include, but are not limited to, bacterial cells, such as *E*. *coli,* Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art. The thus produced first and/or second molecule may then be introduced into the cell in step (a) of the method of the invention. Alternatively, the expression of the above described nucleic acid molecule encoding said fusion protein and being in expressible form, e.g., as part of an expression vector, can be introduced into the cell so that the first and/or second molecule can be expressed within the cell. It is to be understood that the nucleic acid in expressible form referred to in step (a)(i) and (a)(ii) of the method of the invention may not necessarily mean that two separate nucleic acids need be introduced into the cell to achieve expression of the first and second molecule within the cell, but that both molecules may be encoded by one nucleic acid molecule that is introduced into the cell.

The recombinantly expressed molecule may contain additional amino acid residues in order to increase the stability or to modify the targeting of the protein. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the molecules to be used in the method of the invention. The addition of peptide moieties to polypeptides to engender secretion or excretion, to improve stability and to facilitate purification, among others, are familiar and routine techniques in the art. Fusion proteins may comprise a heterologous region from immunoglobulin that is useful to stabilize and purify proteins. For example, EP-A-0 464 533 discloses fusion proteins comprising various portions of constant regions of immunoglobulin molecules together with another human protein or part thereof. In many cases, the Fc part in a fusion protein is thoroughly advantageous for use in therapy and diagnosis and thus results, for example, in improved pharmacokinetic properties (EP-A 0 232 262). It is to be understood, however, that the Fc portion is advantageously no part of the fusion protein obtainable in accordance with the invention. It is further understood that any of the aforementioned modifications to the fusion proteins described herein above does not compromise their functionality in the method of the invention. The fusion proteins, i.e. the molecules of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography and/or hydroxylapatite chromatography. Most preferably, fast protein liquid chromatography (FPLC) is employed for purification, however, high performance liquid chromatography ("HPLC") may also be used.

In accordance with the present invention it is envisaged that the fusion protein, i.e. the first and/or second molecule is expressed from a viral expression system or an expression system involving a phage. A number of such expression systems have been described in the art (see e.g. Hoogenboom et al., 1998; Pluckthun, 1994; Verma et al, 1998). Particularly preferred are expression systems which involve surface exposure of the amino acid residues forming the variable region, since this allows performing selection based on the interaction of said variable region with an antigen of interest.

In case the detection moiety is not a protein it may be chemically linked to the single-domain antibody. Appropriate coupling strategies include amine coupling via N-hydroxysuccinimid (NHS)/ 1-ethyl-3-(3-dimethylaminopropyl)carbodiimid (EDC) esterification e.g. at lysine residues and/or thiol coupling via NHS/EDC and (2-(2-pyridinyldithio)ethaneamine hydrochloride (PDEA) e.g. at cysteine residues.

The first and second single-domain antibody as part of the first and second molecule, respectively, each bind to a portion on the target that is distinct from each other so that upon simultaneous binding to their respective binding portion, the first and second single-domain antibody (and thus the first and second molecule) are spatially separated from each other. The term "binding" as used in this context refers to specific binding. Specific binding means epitope specific binding and is specific, when a single-domain antibody statistically only binds to a particular epitope and does not or essentially not bind to an unrelated epitope. In other words, the single-domain antibody does not display cross-reactivity or does essentially not display cross-reactivity with other epitopes than the one it was generated against. In this context, the term "essentially" is meant to refer to an insignificant or negligible cross-reactivity. The insignificance or negligibility can be based on functional or quantitative parameters. For example, only a minimal amount of cross-reactivity occurs with a different epitope and/or cross-reactivity occurs, however, with a different epitope that is present in insignificant amounts and/or the binding of the single-domain to the different epitope is of no consequence (e.g., if the single-domain antibody of the other molecule of the method of the invention does not display cross-reactivity with a different epitope.). Preferably, the single-domain antibodies do not display cross-reactivity. It follows from the foregoing that the "first portion" and the "second portion" bound by the first and second single domain antibody, respectively, comprise or consist of essentially unique epitopes different from each other within the cell to serve as (specific) binding sites for the first and second molecule. As a consequence, the first and second portion of the target is spatially distinct, thereby allowing the simultaneous binding of the first and second molecule to the target. Only in specific scenarios, such as to detect and quantify, e.g., dimerization or oligomerization, the first and second single-domain antibody may bind to the same epitopes on each dimerizing or oligomerizing molecule, wherein said epitope is to occur only once on each dimerizing or oligomerizing molecule. It can be expected that there is an even amount of first molecule and second molecule pairs bound on, e.g., the dimerizing molecules, versus two first or two second molecules bound thereto from which no detectable fluorescent signal can be generated.

The choice of the first and second (binding) portion is essential for the method of the invention. Only when the first and second detection moiety are brought into spatial proximity upon binding of the first and second molecule to their respective portion on the target, a detectable fluorescent signal can be generated under suitable conditions. Therefore, the choice of the first and second portion must be such that upon binding of the first and second molecule to their respective binding sites they are in spatial proximity allowing the detection of a fluorescent signal occurring as a consequence of fluorescent Förster resonance energy transfer (FRET) or bimolecular fluorescence complementation (BiFC) under conditions suitable for generating said detectable fluorescent signal emitted by the combination of first and second molecule bound to the target as a consequence of FRET or BiFC occurring. It is understood by the skilled person that the spatial proximity necessary to elicit said detectable fluorescent signal under suitable conditions is dictated by the choice of the first and second detection moiety interacting with each other. Thus, the spatial proximity may vary depending on the specific detection moiety setup chosen for first and the second molecule to enable the occurrence of FRET or BiFC for eliciting a detectable fluorescent signal under suitable conditions. Independent from the choice of the detection moieties in the case FRET is to occur for eliciting a detectable fluorescent signal, the spatial proximity between the first and the second detection moiety upon binding of the first and the second molecule to their respective portion on the target is in the range of 1 to 10 nm. Independent from the choice of the detection moieties in the case BiFC is to occur for eliciting a detectable fluorescent signal, the spatial proximity between the first and the second detection moiety upon binding of the first and the second molecule to their respective portion on the target is such that a complementation interaction between the two halfs of the fluorescent protein chosen for BiFC each represented by one detection moiety can occur. It is understood that the spatial proximity varies depending on the fluorescent protein chosen for BiFC. The person skilled in the art is in the position to select suitable fluorescent proteins for BiFC in dependence of the specific experimental setup and the target to be detected by the method of the invention. The skilled person is in the position to identify suitable first and second (binding) portions and generate single-domain antibodies specifically binding to said first and second portions according to methods well-known in the art in dependence from the choice of the detection moieties or vice versa (cf. e.g., Arbabi-Ghahroudi et al., 1997; Strokappe et al., 2012).

The requirement of the combination of first and second detection moiety to allow the occurrence of fluorescent moiety Förster resonance energy transfer or bimolecular fluorescence complementation when in spatial proximity dictates the nature of said detection moieties being part of the first and second molecule to be fluorescent molecules, i.e. fluorphores. Briefly, the term "fluorescent moiety Förster resonance energy transfer" (abbreviated "FRET") relates to the physical mechanism of nonradiative energy transfer through dipole-dipole coupling from an excited donor fluorophore to an acceptor fluorophore, when donor chromophore and acceptor chromophore reside in close spatial proximity, typically less than 10 nm (Jares-Erijman and Jovin, 2003). In live cell imaging, fluorescent proteins are most preferably used as donor and acceptor fluorophores, with CFP/YFP and GFP/mCherry being common FRET pairs (Piston and Kremers, 2007). The skilled person is aware of various compounds that can be used in combination so that FRET is to occur between said given compounds. In the case the detection moieties are chosen in order for FRET to occur when in spatial proximity, either the first or the second detection moiety can be the donor detection moiety. The term "bimolecular fluorescence complementation" (abbreviated "BiFC") relates to the functional reconstitution of fluorescent protein fragments into their native conformation, when these fragments are specifically brought in close spatial proximity (Hu et al., 2002). The most commonly used fluorescent protein fragments are derived from eYFP, eCFP and Venus. The most common truncation sites for FP fragmentation are at amino acid position 155 or 173 (Kerppola, 2006). A variety of fluorophores such as, e.g. fluorescent proteins and non-protein organic fluorophores can be used as detection moieties. Fluorophores are molecules whose excitation with light having a wavelength in the range suitable for exciting a given fluorophore results in the emission of detectable energy. The skilled person is in the position to select a first and a second detection moiety from the multitude of available fluorophores (see below) between which FRET can occur. Also, he is in the position to fragment a given fluorescent protein into two molecules that can be used as first and second detection moiety so that BiFC can occur as, e.g., described in Kerppola, 2006.

The designation "first" and "second" as used in the context of the molecules, the portions, the targets and the detection moieties is not meant to refer to a certain order in a numerical sense or imply a certain structural setup, but only for differentiation purposes, and as such could in principle also be substituted by a different identifier for designation purposes.

The term "single-domain antibody" is well-known in the art to refer to an antibody fragment composed of a single monomeric variable antibody domain and is used herein accordingly. The variable domain of said antibodies comprises three regions of particular sequence variability which are termed hypervariable regions and are denoted HV1, HV2 and HV3. The rest of the V domain shows less sequence variability and the regions adjacent to the hypervariable regions, which are relatively invariant and form the beta-sheet based, barrel-like scaffold of the domain, are termed framework regions (FR1, FR2, FR3 and FR4). The hypervariable regions are formed by loops in the structure of the V domain, which together form the antigen binding site of the immunoglobulin. As the three hypervariable loops essentially constitute the binding site for antigen and determine specificity by forming a surface complementary to the antigen, they are more commonly termed the complementary determining regions, or CDRs, and are denoted CDR1, CDR2 and CDR3. Single-domain antibodies are about 100 to about 130 amino acids long. The term "about" as used in the context of the present invention refers to an average deviation of maximum +/- 10 %, preferably +/-5 %. Preferably, the monomeric domain is a heavy chain variable domain, however, also single-domain antibodies comprising or consisting of a light chain variable domain are within the scope of the invention. The first and second molecule may both either comprise a single-domain antibody made up of heavy chain or light chain variable domains or may each comprise a different variable domain. Single-domain antibodies are typically derived from human, mouse, rat, goat, rabbit or donkey IgG but may also origin from other organisms and/or other immunoglobuline subclasses. Generation of single-domain antibodies typically involves immunization with the target of choice, followed by isolation and subcloning of the gene repertoire encoding variable immunoglobuline (Ig) domains from Ig-producing cells (for example B-lymphocytes), followed by selection and identification using e.g. the phage display method as described, e.g., in Holt et al., 2003. Alternatively, single-domain antibodies may be derived from heavy-chain antibodies found in Camelids as described in Arbabi-Gharhoudi et al., 1997, or Chondrichthyes (cartilaginous fishes). The single domain antibodies originating from Camelids are known as V_{H}H fragments and those generated from Chondrichthyes heavy chain antibodies are known as V_{NAR} fragments. Single-domain antibodies may be optimized, e.g., to improve their stability towards enzymes etc. In order to improve antigen recognition, it may be desirable to substitute one or more amino acid residues. Preferably, this substitution will be performed in CDR1, CDR2 and/or CDR3. A random approach or a selective approach may be chosen to generate these substitution mutants. Sometimes it may be necessary to exchange further amino acid residues outside of the CDRs in order to generate antibodies with sufficient stability or sufficient affinity to a given antigen. In contrast to V_{H} domains of conventional antibodies, the V_{H}H fragments of, e.g., camel heavy chain antibodies are expressed efficiently as soluble non-aggregating recombinant proteins due to their unique hydrophilic substitutions in framework 2: V37F/Y, G44E, L45R. This shows the importance of the amino acid residues outside the CDRs for the stability and solubility of the fragments. A directed mutagenesis of single or multiple amino acid residues could lead to an improvement of the stability or solubility. Also humanization of the single-domain antibodies can be effected by the skilled person according to state of the art procedures if desired (summarized in Muyldermans, 2001).

The conditions for generating a detectable fluorescent signal from the combination of first and second detection moiety when in spatial proximity that the cell is subjected to in step (b) of the method of the invention conceivably depend on the nature of the detection moieties. Thus, the term "suitable" in the context of the term "conditions" is a placeholder and refers to the varying conditions that can be applied to generate a detectable fluorescent signal from the varying combinations of detectable moieties. Generally, conditions are such that the cells are radiated with light of a wavelength suitable to excite the donor detection moiety in case the detection moieties are chosen so that FRET can occur or to excite the functionally complemented detection moieties in case the detection moieties are chosen so that BiFC can occur.

As the skilled person is aware, there are various methods for analysing whether FRET or BiFC occurs in the cell upon subjecting the cell to conditions suitable for generating said detectable fluorescent signal in step (b) of the method of the invention. If the detection moieties are chosen so that FRET can occur, one way is to analyse whether a fluorescent signal emitted from the acceptor detection moiety can be detected upon excitation of the donor detection moiety (Sensitized Emission). Moreover, acceptor photobleaching (APB) may be applied to analyse donor fluorescence dequenching/increase due to FRET. Alternatively, one may use fluorescence-lifetime imaging microscopy, where the lifetime of fluorophores (the average time the fluorophore stays in its excited state before it emits a photon and drops to the ground state) is measured, e.g. using a time-correlated single-photon counting (TCSPC) device. When FRET occurs between a donor and an acceptor fluorophore, the donor fluorophore lifetime is decreased. If the detection moieties are chosen so that BiFC can occur, one can analyse whether a fluorescent signal is emitted from the combined first and second detection moiety. As is known the person skilled in the art, various methods exist to perform the above mentioned analyses of step (c) of the method the invention. Generally, they will involve a means for detecting a fluorescent signal emitted by the combination of the first and second detection moiety upon excitation. Preferably, imaging techniques are used that allow the detection of said detectable fluorescent signal within the analyzed cell such as, e.g., fluorescence microscopy, fluorescence spectroscopy and flow cytometry techniques. More preferably, fluorescence microscopy, e.g., epifluorescence or confocal microscopy is used which allows visualizing the subcellular location of said detectable fluorescent signal within the analyzed cell. As is understood by the skilled person, the detection methods referred to herein above are also suitable to perform a quantification of the target in case its presence has been detected.

In accordance with the invention, the first and second molecule need not be identical in their setup, but may independently from each other have, e.g., single-domain antibodies from different origin, fluorophores from different groups, be introduced differently, as long as the requirements described herein above to be essential for the method of the invention are met.

The term "nucleic acid" as used throughout the specification of the present invention refers to DNA or RNA, including genomic DNA, cDNA, mRNA, hnRNA, and synthetic derivatives thereof.

The skilled person is in the position to adapt, vary and/or validate (e.g., by using suitable negative and positive controls) the method of the invention should it be necessary by using standard techniques that may involve a try-and-error-approach. In this regard, the method of the invention may be adapted so that the presence or absence of more than one target can be detected and, optionally, be quantified, within a cell by providing two or more first and second molecule pairs as defied herein above which can be differentially detected, e.g., by means of using different detection moieties or different detection methods. Therefore, the method of the invention allows the detection of the presence or absence of multiple such as, e.g., two, three, four, five, six, seven, eight, nine, ten or more targets simultaneously within one cell. Thus, in a preferred embodiment, the method of the invention comprises the introduction of one or more further first and second molecule pairs as defined herein above but each directed to a different target.

As is the case with any detection method, also the detection of the presence or absence of a given target by the method of the invention can be influenced by a variety of factors (e.g., the choice of target, binding portion, detection moiety, or cell) and, thus, the result may or may not be biased to a certain extent in this regard. In other words, while according to the results obtained when executing the method of the invention a given target is determined to be absent from a cell, it may nevertheless be present at levels below the detection threshold. It is technical routine to determine the detection thresholds for detection methods in dependence of various experimental parameters such as, e.g., the detection moiety, target or cell used.

The method of the present invention allows for the first time to detect and advantageously to also simultaneously quantify a specific target in living cells with the same method and without the need to manipulate the target, e.g., by fusing it with detectable moieties or otherwise modifying an endogenously occurring target within the cell. Thus, the method allows to dynamically analyze the presence or absence and/or the amount of a target within a cell with minimal to no manipulation of the target of interest. The method of the invention closes an important analytical gap in live cell analysis. The method of the invention may, thus, be particularly relevant in situations where it remained previously impossible to detect and/or determine the presence or absence and/or the amount of a natural, unmodified target of interest in living cells, possibly over a period of time. Generally, the method of the invention may be applied to all intracellular target structures. Specific areas that would particularly benefit from the method of the invention include the dynamic detection of factors (proteinaceous and non-proteinaceous factors or combinations thereof) that are involved in cell cycle regulation (e.g. cyclin proteins), the dynamic detection of factors (proteinaceous and non-proteinaceous factors or combinations thereof) that are involved in regulation of gene expression (e.g. epigenetic factors such as DNA-methyltransferases, histon-methyltransferases, histon-acetylases, histon-deacetylases etc., DNA methylation, histon-methylation, histon acetylation), the dynamic detection of factors (proteinaceous and non-proteinaceous factors or combinations thereof) that are involved in DNA-repair mechanisms (e.g. DNA ligases, DNA glycosylases, DNA endonucleases and DNA break point markers such as γH2AX), the dynamic detection of pathogenic factors, (e.g. viral and/or cancerous factors). Moreover, the method of the invention is less laborious and thus cost-effective than other detection and quantification approaches in living cells as the steps of modifying the target to be analysed are omitted.

In a preferred embodiment, the method of the invention further comprises the step of quantifying the target detected.

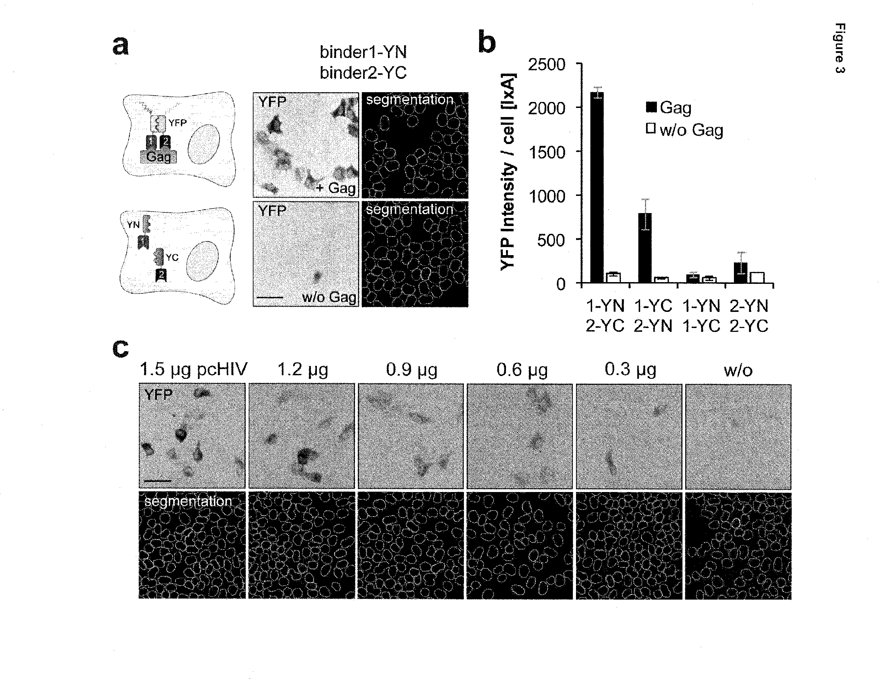

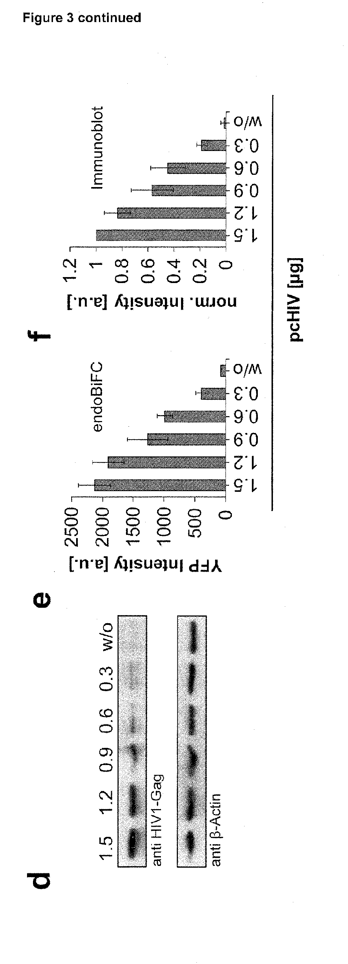

The skilled person is aware of various approaches to quantify the target should the presence of the target be detected on the basis of the respective analysis method used that may or may not involve the use of controls, i.e. cells in which no FRET or no BiFC occurs. For example, and in case FRET is used for target detection, acceptor photobleaching (APB) can be performed. When FRET occurs between donor and acceptor detection moieties when in spatial proximity, excitation of the donor detection moiety results in quenched donor detection moiety emission, since a significant amount of absorbed energy is used for acceptor detection moiety excitation. Specific photobleaching of acceptor detection moieties thus leads to de-quenched, increased donor detection moiety fluorescence. Furthermore, and in case BiFC is used for detection, the total fluorescence intensity which is dependent on the target to be detected and can be measured in large cell populations or single cells, proportionally and directly relates to the amount of target to be detected (s. Figure 3).

In another preferred embodiment of the method of the invention, said first and second single-domain antibody are each independently selected from a V_{H}H fragment derived from a camelid antibody or a V_{NAR} fragment from a chondrichthyes antibody.

Camelids (Bactrian camels, dromedaries, Ilamas (including the species Ilama, guanaco and alpaca) and vicugna) produce functional antibodies devoid of light chains of which the single N-terminal domain is capable of binding an antigen. These single-domain antibodies fragments are termed V_{H}H fragments (also known under the trademark name Nanobodies® and referred to herein) and are capable to bind to an antigen (epitope) without requiring domain pairing. They are well-expressed in microorganisms and have a comparatively high stability and solubility (Harmsen, 2007). Methods to isolate antigen-specific V_{H}Hs from immune (Arbabi-Ghahroudi et al., 1997; Van der Linden et al., 2000), non-immune (Tanha et al., 2002; Yau et al., 2003; Verheesen et al., 2006), or semisynthetic (Goldman et al., 2006) libraries using phage, yeast, or ribosome display are well established. While the use of conventional antibody fragments is also encompassed by the invention, the use of single-domain antibody fragments as referred to in this embodiment is preferred due to, e.g., their small size (allowing proximity targeting), functionality in living cells, rapid tissue penetration, fast clearance (resulting from small size), recognition of hidden antigenic sites (resulting from small size and extended flexible CDR3), high solubility and high stability.

The animal class chondrichthyes is well-known by the person skilled in the art and is also referred to as cartilaginous fish. Initially, single-domain antibodies have been isolated from the nurse shark, *Ginglymostoma cirratum* (Greenberg et al., 1995) and wobbegong shark, *Orectolobus maculates,* which may be used in accordance with the present invention. As camelid single-domain antibody fragments can be isolated using libraries, this has recently also been shown to be possible with shark single-domain antibody fragments (Shao et al., 2007).

In a further preferred embodiment of the method of the invention, said first and second detection moieties are independently from each other selected from the group consisting of fluorescent proteins, quantum dots, and non-protein organic fluorophores, if FRET is to occur; or said first and second fluorescent moieties are complementing fragments of fluorescent proteins selected from the group consisting of YFP, CFP, GFP and Venus.

As mentioned herein above, a variety of fluorophores exist that can be used as detection moieties in the method of the present invention. One possibility is to use a pair of fluorescent proteins that can act as donor and acceptor detection moiety pair so that FRET can occur between the first and second molecule upon binding to the first and second portion of the target. The use of fluorescent proteins as detection moieties has the advantage that the first and second molecule can be prepared as fusion protein (as detailed herein above) and, thus, be introduced into the cell as a nucleic acid in expressible form from which the molecules can then be expressed. One group of fluorescent proteins includes Green Fluorescent Protein isolated from *Aequorea victoria* (GFP), as well as a number of GFP variants, such as cyan fluorescent protein, blue fluorescent protein, yellow fluorescent protein, etc. (Zimmer, 2002; Zhang et al., 2002). A number of color shift mutants of GFP have been developed and may be used as detection moieties. These color-shift GFP mutants have emission colors blue to yellow-green, increased brightness, and photostability (Tsien, 1998). One such GFP mutant, termed the Enhanced Yellow Fluorescent Protein, displays an emission maximum at 529 nm. Additional GPF-based variants having modified excitation and emission spectra (Tsien et al., U.S. Patent Appn. 200201231 13A1), enhanced fluorescence intensity and thermal tolerance (Thastrup et al., U.S. Patent Appn. 20020107362A1; Bjorn et al., U.S. Patent Appn. 20020177189A1), and chromophore formation under reduced oxygen levels (Fisher, U.S. Patent No. 6,414,119) have also been described. Recently, GFPs from the anthozoans *Renilla reniformis* and *Renilla kollikeri* were described (Ward et al., U.S. Patent Appn. 20030013849).

Another group of such fluorescent proteins includes the fluorescent proteins isolated from anthozoans, including without limitation the red fluorescent protein isolated from *Discosoma* species of coral, DsRed (Matz et al., 1999; see, e.g., accession number AF168419 version AF16849.2). DsRed and the other anthozoan fluorescent proteins share only about 26-30% amino acid sequence identity to the wild-type GFP from *Aequorea victoria,* yet all the crucial motifs are conserved, indicating the formation of the 11-stranded beta-barrel structure characteristic of GFP. The crystal structure of DsRed has also been solved, and shows conservation of the 11-stranded beta-barrel structure of GFP MMDB Id: 5742.

A number of mutants of the longer wavelength red fluorescent protein DsRed have also been described, and similarly, may be employed as detection moieties. For example, recently described DsRed mutants with emission spectra shifted further to the red may be employed in the practice of the invention (Wiehler et al., 2001; Terskikh et al., 2000; Baird et al., 2000).

An increasingly large number of other fluorescent proteins from a number of ocean life forms have recently been described, and the Protein Data Bank currently lists a number of GFP and GFP mutant crystal structures, as well as the crystal structures of various GFP analogs. Related fluorescent proteins with structures inferred to be similar to GFP from corals, sea pens, sea squirts, and sea anemones have been described, and may be used in detection moieties (for reviews, see Zimmer, 2002; Zhang et al., 2002).

Fluorescent proteins from *Anemonia majano, Zoanthus sp., Discosoma striata, Discosoma sp.* and *Clavularia sp.* have also been reported (Matz et al., supra). A fluorescent protein cloned from the stony coral species, *Trachyphyllia geoffroyi,* has been reported to emit green, yellow, and red light, and to convert from green light to red light emission upon exposure to UV light (Ando et al., 2002). Recently described fluorescent proteins from sea anemones include green and orange fluorescent proteins cloned from *Anemonia sulcata* (Wiedenmann et al., 2000), a naturally enhanced green fluorescent protein cloned from the tentacles of *Heteractis magnifica* (Hongbin et al., 2003), and a generally non-fluorescent purple chromoprotein displaying weak red fluorescence cloned from *Anemonia sulcata,* and a mutant thereof displaying far-red shift emission spectra (595nm) (Lukyanov et al., 2000).

Additionally, another class of GFP-related proteins having chromophoric and fluorescent properties has been described. One such group of coral-derived proteins, the pocilloporins, exhibit a broad range of spectral and fluorescent characteristics (Dove, 1999; Dove et al., 2001). The purification and crystallization of the pocilloporin Rtms5 from the reef-building coral *Montipora efflorescens* has been described (Beddoe et al., 2003). Rtms5 is deep blue in colour, yet is weakly fluorescent. However, it has been reported that Rtms5, as well as other chromoproteins with sequence homology to Rtms5, can be interconverted to a far-red fluorescent protein via single amino acid substitutions (Beddoe et al., 2003, supra; Bulina et al., 2002; Lukyanov et al., 2000, supra).

Various other coral-derived chromoproteins closely related to the pocilloporins are also known (see, for example, Lukyanov et al. 2000; Gurskaya et al., 2001). Any of the fluorescent proteins or fluorescent fragments thereof may be used in accordance with the teaching of the present invention. Further examples of fluorescent proteins are GFP form Renilla reniformis, mKO from Fungia concinna, Azami Green from Galaxeidae or cOFP from Cerianthus. Fragments of the fluorescent proteins for use as detection moieties if FRET as well as BiFC is to occur are functional fragments, i.e. capable of being excited and emitting energy as a consequence of said excitation.

A first and a second detection moiety between which FRET can occur is to be selected based on the emission wavelength of one fluorophore and the excitation wavelength of another fluorophore. Exemplary FRET pairs that can be used in accordance with the method of the invention can be found in the following table:

| **Donor fluorophore** | **Acceptor fluorophore** |
|---|---|
| **Fluorescent protein FRET pairs** | |
| GFP | mRFP |
| GFP | mCherry |
| CFP | YFP |
| Cerulean | Venus |

| **Organic dye FRET pairs** | |
|---|---|
| Cy2 | Cy3 |
| Cy3 | Cy5 |
| Alexa Fluor 488 | Alexa Fluor 546 |
| Alexa Fluor 488 | Alexa Fluor 610 |
| Alexa Fluor 647 | Alexa Fluor 680 |
| Alexa Fluor 647 | Alexa Fluor 700 |
| Atto 488 | Atto 590 |
| Atto 550 | Atto 655 |
| Fluorescein | Tetramethylrhodamine |

Other potential fluorophores that can be used as detection moieties are quantum dots (QD) and non-protein organic dyes. QDs are inorganic semiconductor nanocrystals (e.g. CdSe, ZnS, CdS and InP QDs). Their spectral properties can be size- and/or material dependent and may be tuned accordingly. QDs can be conjugated to biomolecules such as antibodies for cellular applications (Bruchez et al., 1998; Goldman et al., 2002; Jaiswal et al., 2003). Moreover, a broad variety of organic fluorescence dyes (e.g. Alexa Fluor® dyes, Atto dyes, cyanine dyes) is available to specifically label biomolecules. Their organic nature enables simple labeling via conjugation chemistry. Both classes of fluorophores, QDs and organic dyes, can be attached to biomolecules e.g. single-domain antibodies *in vitro* for functional delivery into living cells.

Bimolecular fluorescence complementation (BiFC) has been developed as a sensitive method to visualize and detect protein-protein interactions. The technique is based on the concept that non-fluorescent fragments of fluorescent proteins may associate to form a fluorescent complex, when brought in spatial proximity. BiFC has been adapted using a variety of fluorescent proteins, including GFP, YFP, CFP and Venus (Kerppola, 2006). Fragmentation of a given fluorescent protein into two molecules that can be used as first and second detection moiety so that BiFC can occur is possible according to methods known in the art and referred to herein. Preferably, the fluorescent proteins are selected from YFP, CFP, GFP and Venus whose fragmentation has been described in Kerppola, 2006. Exemplary BiFC fragments that can be used in accordance with the method of the invention can be found in the following table:

| **N-terminal fragment** | **C-terminal fragment** |
|---|---|
| YFP 1-154 (YN155) | YFP 155-238 (YC155) |
| CFP 1-154 (CN155) | CFP 155-238 (CC155) |
| GFP 1-154 (GN155) | GFP 155-238 (GC155) |
| Venus 1-154 (VN155) | Venus 155-238 (VC155) |
| mCherry 1-159 (MN159) | mCherry 160-237 (MC160) |

In another preferred embodiment of the method of the invention, the target comprises a first and a second binding partner and the first portion bound by the first single-domain antibody is present only on the first binding partner and the second portion bound by the second single-domain antibody is present only on the second binding partner, and wherein said detectable fluorescent signal is generated in step (b) of claim 1 upon binding of the first binding partner to the second binding partner.

This embodiment relates to the detection of the presence or the absence of a complex formed by known binding partners. In other words, the method detects whether two binding partners are associated with each other or not. Thus, the target in this embodiment comprises or consists of two entities, i.e. a first and a second binding partner, known to bind to each other. Conceivably, only when it is known that two molecules bind to each other, it will be possible to determine suitable first and second portions on the first and second binding partner, respectively, for generating single-domain antibodies binding thereto as part of a first and second molecule pair. Only when the binding partners are associated, i.e. have formed a complex, the first and second molecule when bound to their respective portion on a binding partner can be used to generate a detectable fluorescent signal. Thus, this embodiment provides the possibility to detect and quantify molecule interactions in living cells without the need to modify the molecules whose interaction is to be detected and quantified. As such, the method of this embodiment may be used in a variety of situations and applications such as, e.g., high-throughput screening (HTS) for protein-protein interactions and/or drug-screening.

In a further preferred embodiment, the target comprises a first and a second binding partner and the first portion bound by the first single-domain antibody is present on the first binding partner and the second portion bound by the first single-domain antibody is present on the second binding partner, wherein said first and second portion are the same, and wherein said detectable fluorescent signal is generated in step (b) of claim 1 upon binding of the first binding partner to the second binding partner.

In an alternative to the previous embodiment, and applicable only to detect and quantify the binding of two or more of the same binding partners (oligomerization, dimerization, etc.), in this embodiment the first and second portion may be the same. As a consequence, the single-domain antibody of the first and second molecule have the same target specificity, i.e. bind to the same epitope.

In an alternative preferred embodiment of the method of the invention, said first and second binding portions are located on the target to be detected in a manner so that said detectable fluorescent signal is generated in step (b) of claim 1 upon a conformational change of the target.

This embodiment is directed at determining the presence and absence of a conformational change of a target. As such, both or one of the portions on the target bound by the respective molecule may only become accessible upon the conformational change so that a fluorescent signal can be generated and detected, or ― vice versa ― may become inaccessible so that no fluorescent signal can be generated and detected. A further possible setup is to choose a first and a second portion on the target that upon conformational change of the target bring said portions into close spatial proximity. As a consequence, and when a first and a second molecule pair is bound to said respective portions on the target, a detectable fluorescent signal is generated when the target changes its conformation. This embodiment may be used in a variety of situations and applications such as, e.g., intracellular signaling (allosteric activation/inactivation), or Receptor-mediated signaling at cellular membranes.

In a further embodiment, the invention relates to a composition comprising a first molecule and a second molecule or one or more nucleic acids encoding said first or second molecule as defined herein above.

As will be appreciated by the skilled person, the first and second molecule or one or more nucleic acids encoding said first or second molecule may be provided together in the form of a composition consisting of or comprising the latter molecules. Preferably, the composition comprises further constituents such as, e.g., conservatives, stabilizers, antibiotics, cell culture media, cell culture media constituents, and/or complexing agents. Also envisaged is the addition of one or more constituents generally used in a method for transducing so as to provide a "ready-to-use" composition for the method of the invention.

In a further embodiment, the invention relates to a kit comprising a first molecule and a second molecule or one or more nucleic acids encoding said first or second molecule as defined herein above or the composition defined herein above and, optionally, instructions for use.

The kit of the invention may contain further ingredients such as components for cell culture media. The kit of the invention can be used for carrying out the method of the invention and could be, *inter alia,* employed in a variety of applications, e.g., in the diagnostic field or as research tool. The parts of the kit of the invention can be packaged individually in vials or other appropriate means depending on the respective ingredient or in combination in suitable containers or multicontainer units. Manufacture of the kit follows preferably standard procedures which are known to the person skilled in the art.

The figures show:

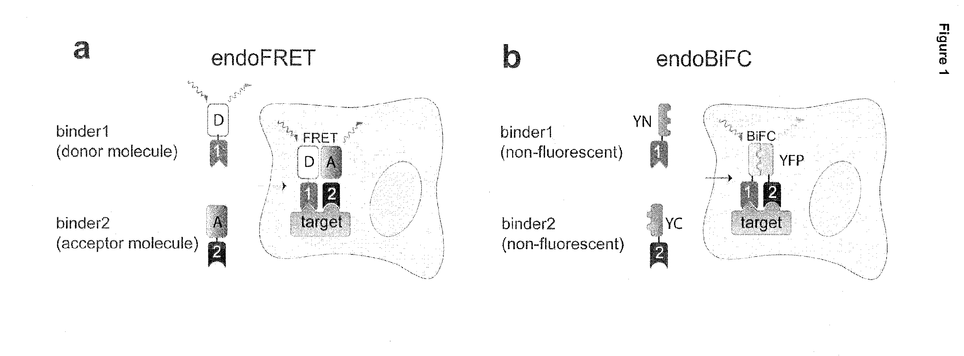

**Figure 1****: Generation of intracellular binders for quantitative protein detection**
   (a) Scheme for endoFRET showing two binders, fused with FPs (donor and acceptor) that recognize distinct, close-by epitopes within an intracellular target structure. Simultaneous antigen binding enables FRET between donor and acceptor. (b) Scheme for endoBiFC. Two binders are fused with non-fluorescent FP fragments that functionally complement upon simultaneous antigen binding.

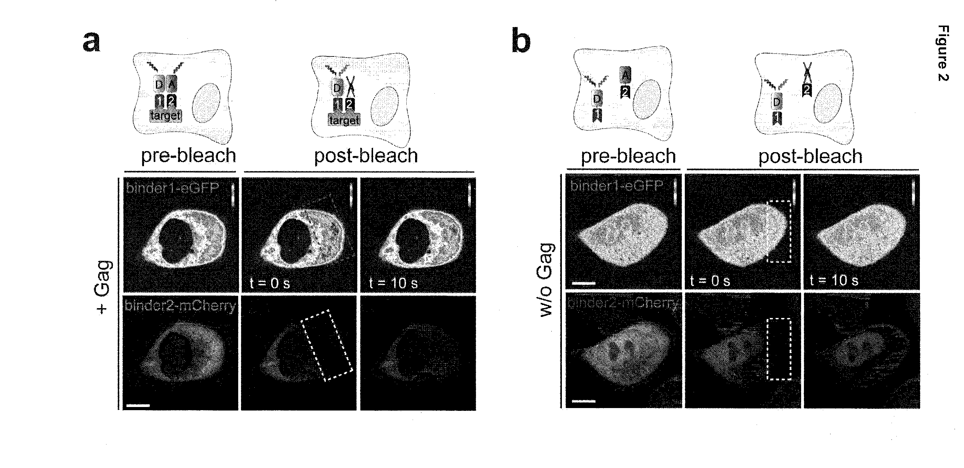

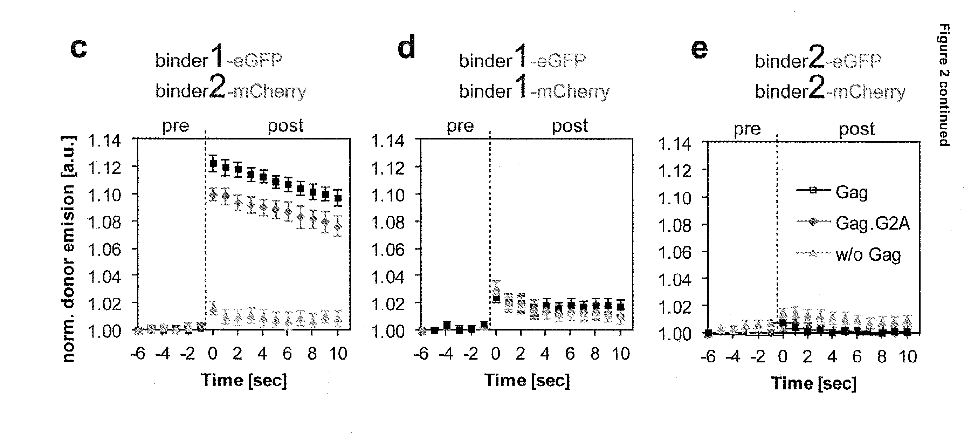

**Figure 2****: Intracellular protein detection with endoFRET**
   (a) The strategy for detecting HIV-1 Gag with endoFRET is outlined. A Hela-Kyoto cell, expressing binder1-eGFP (top), binder2-mCherry (bottom) and HIV-1 Gag, as indicated by the cytoplasmic localization of the specific binders. eGFP (donor) fluorescence intensity is displayed in heatmap mode. Pre- and post-bleach time frames are depicted on the left and on the right, respectively. The bleached region is marked in dashed lines. Scale bar is 10 µm. (b) Experimental setup as in (a) except that Gag is not expressed. (c-e) Donor emission intensities of hetero- and homoepitopic binder pairs in the presence of Gag, a non-myristoylated mutant Gag.G2A or in absence of Gag. Initial fluorescence intensity was set to 1.
**Figure 3****: Quantitative detection of proteins with endoBiFC**
   **(a)** Shown are HeLa-Kyoto cells, expressing binder1-YN and binder2-YC together with HIV-1 Gag (top) or without Gag (bottom). Gag is visualized by cytoplasmic fluorescence of complemented YFP. Scale bar is 100 µm **(b)** Quantitative high content analysis. Integrated YFP intensity (n>15000 cells) as a function of different hetero-epitopic and homo-epitopic binder combinations with or without Gag reveals the specificity of endoBiFC. Error bars represent standard deviations from 3 independent transfection experiments. **(c)** endoBiFC signal as a function of pcHIV DNA amount used for transfection. Scale bar is 100 µm. **(d)** Immunoblot analysis with a polyclonal antibody against HIV-1 Gag from the very same transfection used for endoBiFC imaging. β-Actin was detected as loading control. **(e)** Automated quantification of complemented YFP signal as shown in (c). Errors are standard deviations from three independent transfection experiments. **(f)** Densitometric quantification of western blot signals as shown in (d). Errors are standard deviations from three independent transfection experiments

The examples illustrate the invention:

### Example 1: Material and Methods

### Plasmids and proteins

For intracellular expression of binder1 and binder2 in mammalian cells, translational fusions with eGFP and mCherry were cloned using BgIII/HindIII sites in peGFP-N1 and AsiSI/NotL sites in a modified peYFP-N1, where eYFP was replaced by mCherry. For bimolecular fluorescence complementation experiments, binder1 and binder2 were each fused to N- and C-terminal eYFP fragments (YN₁₋₁₅₄; YC₁₅₅₋₂₃₈), using BamHI/NotI restriction sites in peYFP-N1. Subcellular interaction with binder1 and binder2 was analyzed using a non-infectious plasmid pcHIV encoding untagged HIV-1 (Lampe et al., 2007; Muller et al., 2004) and pcHIV_{Gag.G2A}, where the Gag myristoylation site Gly2 was replaced with Ala by site-directed mutagenesis. All plasmids were checked by DNA sequence analysis.

### Cells and transfection

HeLa-Kyoto cells were maintained in DMEM supplemented with 10% fetal calf serum and gentamycin at 50 µg/ml (PAA). Transfection was performed with Lipofectamine (Invitrogen) according to the manufacturers' instructions. For live cell imaging, cells were seeded in ibidi 8-well slides (Ibidi). During image acquisition, cells were maintained in phenol red-free HEPES buffered DMEM (PAA) at 37°C and supplied with 5% CO₂. For immunoblot analysis and high content imaging, ~10⁷ cells were seeded in p100 dishes format supplemented gridded 18x18 mm coverslips. 24 h post transfection, cells were fixed for 15 min using 3,7% PFA solution in PBS (pH 7.4), permeablized with 0.5% Triton X100/PBST, DAPI-stained and mounted on object slides in Vectashield anti-fading reagent (Vector Laboratories). For FRET measurements, cells were transfected with equal amounts of binder1-eGFP, binder2-mCherry together with pcHIV, pcHIV_{Gag.G2A} or control DNA (0.13 µg DNA each). For BiFC imaging, cells were transfected with 1:1:1 DNA amounts. For differential Gag expression, decreasing pcHIV DNA amounts (1.5; 1.2; 0.9; 0.6; 0.3 µg) were transfected in p100 dishes; DNA amounts for binder1 and binder2 expression constructs were kept constant (1.5 µg); total DNA amount (4.5 µg) was adjusted with non-related DNA.

### Microscopy and Image Analysis

Images were recorded with an UltraVIEW VoX spinning disc microscope (PerkinElmer), equipped with a 63x / 1.4 NA Plan-Apochromat oil immersion objective and a heated environmental chamber set to 37°C. eGFP and mCherry FPs were excited with a 488 nm and 561 nm solid-state laser line, respectively. For acceptor photobleaching experiments, a region of interest was selected (typically covering ~1/3 of the cell) and mCherry molecules were selectively bleached (10 iterations), using the 561 nm laser line at maximum power eGFP and mCherry image series were recorded before and after bleaching (Five pre-bleach frames; 10 post-bleach frames; one second intervals). Quantitative analysis was performed using ImageJ. Mean fluorescence intensity within a bleached region of interest was normalized against background and an unbleached region within the same cell. FRET efficiency was determined according to E= 1 ― I_{eGFP}-ₚᵣₑ/I_{eGFP-post}, where I_{eGFP}-ₚᵣₑ is the last pre-bleach value and I_{eGFP-post} is the first post-bleach value. At least ten cells per experiment were averaged. Mean values and standard error of the mean were calculated using Microsoft Excel. EndoBiFC analysis was performed with an InCell Analyzer 2000 epifluorescence high-throughput microscope (GE Healthcare) equipped with a 40x/0.6 Plan-Fluor air objective. Images were automatically acquired from 100 positions per slide using excitation filters 350/50 (DAPI) and 500/20 (YFP) in combination with emission filters 455/50 (DAPI) and 535/30 (YFP). Quantitative image analysis was performed using InCell Analyzer 1000 Workstation 3.6 analysis software. Cells were segmented and individually identified by nuclear DAPI staining. Cytoplasmic regions were defined by setting a 2 µm collar surrounding individual nuclei. eYFP intensities were separately measured in the nucleus and in the cytoplasm. False-positive BiFC cells with nucleus/cytoplasm intensity ratios > 1.02 were excluded from further analysis. The integrated YFP intensities (BiFC signals) were determined as mean intensity x segmented area. Data were visualized using Microsoft Excel.

### Immunoblot analysis

~10⁷ HeLa-Kyoto cells were lysed in 200 µl lysis buffer containing 20 mM Tris/Cl pH 7.5, 150 mM NaCl, 0.5 mM EDTA, 0.5% Nonidet P-40, 2mM PMSF and mammalian protease inhibitor cocktail (Serva) for 30 minutes at 4°C with repeated pipetting. Total protein amount was estimated with Pierce 660 nm protein detection reagent (Thermo Scientific) and lysates were accordingly adjusted. 1 % fractions of total lysate were separated by SDS-PAGE and visualized by immunoblot analysis. HIV-1 Gag was detected using a rabbit polyclonal antibody (Abcam) and β-Actin was detected using a mouse monoclonal antibody (Sigma). HRP-coupled anti-rabbit (Jackson Immunoresearch) and anti-mouse (Sigma) secondary antibodies in combination with ECL plus detection reagent (GE Healthcare) were used for visualization. The mean intensities of HIV-1 Gag signals were measured using ImageJ and corrected for lane background and normalized to β-Actin.

### Example 2: Results

To generate a FRET-based reporter system for quantitative protein detection, binder1 and binder2 were fused, which bind different, close-by epitopes within the HIV-1 Capsid protein (CA), with eGFP and mCherry at their respective C-termini. Then, their antigen binding properties in live cells were tested. When expressed in HeLa-Kyoto cells, both binders showed diffuse distribution throughout the cells with slight enrichment in the nucleus. To produce the HIV CA antigen in live cells, cells were transfected with pcHIV, a noninfectious construct (Lampe et al., 2007) that carries almost the entire HIV genome. CA is synthesized as an internal part of the viral structural Gag polyprotein, which is expressed in the cytoplasm and assembles viral particles at the plasma membrane. Upon co-transfection with pcHIV, both binders relocated to the cytoplasm, strongly indicating that they recognize their designated epitopes in live cells. It was reasoned that simultaneous epitope recognition by binder1 and binder2 of the same CA moiety should lead to an increase of FRET efficiency between the attached eGFP (donor) and mCherry (acceptor). To monitor FRET, acceptor photobleaching experiments were performed, where bleaching of the acceptor mCherry leads to increased fluorescence of the donor GFP allowing quantification of FRET efficiency. Expression of HIV-1 Gag in HeLa Kyoto cells resulted in significant donor dequenching and a FRET efficiency of about 11%. In the absence of HIV-1 Gag, the FRET efficiency dropped below 2%. A positive control with mCherry fused to eGFP gave 22% FRET efficiency. These results show that the combination of these two CA-specific binders yields an antigen-dependent FRET signal in live cells.

To rule out possible FRET artefacts owing to Gag assembly (Ono et al., 2000), several control experiments were performed. A Gag point mutant (Gag.G2A) that cannot be myristoylated and thus cannot properly bind to the membrane, where Gag assembly occurs (Bryant and Ratner, 1990; Jouvenet et al., 2009), revealed 9% FRET efficiency, similar to wildtype Gag. Furthermore, none of the two homo-epitopic FRET pairs (binder1-eGFP/binder1-mCherry and binder2-eGFP/binder2-mCherry) yielded significant FRET signals, excluding that FRET depended on Gag multimerization. In summary, these results clearly show that the observed FRET is mediated by binding of the nanobodies to adjacent epitopes in CA_{NTD} and CA_{CTD} of the same molecule. Moreover, the determination of FRET efficiency allowed an approximate calculation of the distance between the donor and acceptor. According to the formula *r= R₀(1*/*E-1)*^{*1*/*6*} where the Förster radius *R₀* for eGFP/mCherry is assumed to be 5.1 nm (Albertazzi et al., 2009), an antigen dependent FRET efficiency E of 11 % results in a distance *r* between donor and acceptor chromophores of 7.3 nm, which is in the dimensional range, expected from the crystal structures of the corresponding CA/binder complexes. These results demonstrate that binders recognizing close-by epitopes are well suited to detect endogenous targets by "endoFRET", i.e. the method according to the invention.

The close vicinity of the recognized epitopes is also well suited for bimolecular fluorescence complementation (BiFC) and quantitative read-outs. To test the HIV-specific binders for BiFC, binder1 and binder2 were fused with complementing fragments of eYFP (YN = aa 1-154; YC = aa 155-238) (Hu and Kerppola, 2003) in all four possible combinations. Then, both potentially complementary endoBiFC reporter combinations were transfected in HeLa-Kyoto cells together with or without pcHIV. Fluorescence high-throughput microscopy revealed that both hetero-epitopic binder combinations resulted in a BiFC signal in the presence of Gag, with the binder1-YN/binder2-YC pair being about three times more efficient. No significant BiFC signal was obtained in the absence of Gag. In accordance with the above described endoFRET experiments, none of the homo-epitopic binder pairs resulted in a significant BiFC signal. Finally, it was tested whether endoBiFC can be used to quantify proteins in live cells. Cells with different Gag expression levels were generated by transient transfection with decreasing amounts of pcHIV DNA in the presence of the BiFC reporter pair. Gag protein quantification by immunoblot analysis confirmed decreasing protein levels in these cells. From the very same transfection experiments, cells were analyzed by automated high-content fluorescence microscopy. Images of at least 15000 cells per sample were collected and the BiFC signal was determined by automated image analysis. The comparison of immunoblot signals from cell extracts with the corresponding BiFC signals, demonstrates that endoBiFC can provide a quantitative, optical read-out of protein levels in living cells.

### Reference list:

Albertazzi, L., Arosio, D., Marchetti, L., Ricci, F., and Beltram, F. (2009). Quantitative FRET analysis with the EGFP-mCherry fluorescent protein pair. Photochem Photobiol 85, 287-297.
Bruchez, M., Moronne, M., Gin, P., Weiss, S., and Alivisatos, A.P. (1998). Semiconductor nanocrystals as fluorescent biological labels. Science 281, 2013-2016.
Bryant, M., and Ratner, L. (1990). Myristoylation-dependent replication and assembly of human immunodeficiency virus 1. Proc Natl Acad Sci U S A 87, 523-527.
Deshayes, S., Morris, M.C., Divita, G., and Heitz, F. (2005). Cell-penetrating peptides: tools for intracellular delivery of therapeutics. Cell Mol Life Sci 62, 1839-1849.
Goldman, E.R., Anderson, G.P., Tran, P.T., Mattoussi, H., Charles, P.T., and Mauro, J.M. (2002). Conjugation of luminescent quantum dots with antibodies using an engineered adaptor protein to provide new reagents for fluoroimmunoassays. Anal Chem 74, 841-847.
Hu, C.D., Chinenov, Y., and Kerppola, T.K. (2002). Visualization of interactions among bZip and Rel family proteins in living cells using bimolecular fluorescence complementation. Mol Cell 9, 789-798. Hu, C.D., and Kerppola, T.K. (2003). Simultaneous visualization of multiple protein interactions in living cells using multicolor fluorescence complementation analysis. Nat Biotechnol 21, 539-545. Jaiswal, J.K., Mattoussi, H., Mauro, J.M., and Simon, S.M. (2003). Long-term multiple color imaging of live cells using quantum dot bioconjugates. Nat Biotechnol 21, 47-51.
Jares-Erijman, E.A., and Jovin, T.M. (2003). FRET imaging. Nat Biotechnol 21, 1387-1395. Jouvenet, N., Simon, S.M., and Bieniasz, P.D. (2009). Imaging the interaction of HIV-1 genomes and Gag during assembly of individual viral particles. Proc Natl Acad Sci U S A 106, 19114-19119. Kerppola, T.K. (2006). Design and implementation of bimolecular fluorescence complementation (BiFC) assays for the visualization of protein interactions in living cells. Nat Protoc 1, 1278-1286. Lampe, M., Briggs, J.A., Endress, T., Glass, B., Riegelsberger, S., Krausslich, H.G., Lamb, D.C., Brauchle, C., and Muller, B. (2007). Double-labelled HIV-1 particles for study of virus-cell interaction. Virology 360, 92-104.
Muller, B., Daecke, J., Fackler, O.T., Dittmar, M.T., Zentgraf, H., and Krausslich, H.G. (2004). Construction and characterization of a fluorescently labeled infectious human immunodeficiency virus type 1 derivative. J Virol 78, 10803-10813.
Ono, A., Demirov, D., and Freed, E.O. (2000). Relationship between human immunodeficiency virus type 1 Gag multimerization and membrane binding. J Virol 74, 5142-5150.
Piston, D.W., and Kremers, G.J. (2007). Fluorescent protein FRET: the good, the bad and the ugly. Trends Biochem Sci 32, 407-414.
Strokappe, N., Szynol, A., Aasa-Chapman, M., Gorlani, A., Forsman Quigley, A., Hulsik, D.L., Chen, L., Weiss, R., de Haard, H., and Verrips, T. (2012). Llama antibody fragments recognizing various epitopes of the CD4bs neutralize a broad range of HIV-1 subtypes A, B and C. PLoS One 7, e33298.
Hargus G et al., Proc Natl Acad Sci U S A, 2010, 107:15921-15926, Jaenisch R. and Young R., 2008, Cell 132:567-582
Saha K, and Jaenisch R., 2009, Cell Stem Cell 5:584-595
Murphy et al.1991, Biochem J. 227:277-279
Bebbington et al. 1992, Bio/Technology 10:169-175
Hoogenboom H.R. et al., 1998, Antibody phage display and its applications, Immunotechnology 4:1-20
Pluckthun A, 1994, Escherichia coli producing recombinant antibodies, Bioprocess Technol. 19:233-252
Verma R. et al, 1998, Antibody engineering: comparision of bacterial, yeast, insect and mammalian expression systems, J. Immunol. Methods 216:165-181
Arbabi Ghahroudi et al., FEBS Lett., 414(3):521-526 (1997)
Holt et al., Trends in biotechnology, 21(11):484-490 (2003)
Muyldermans S., 2001, Reviews in Molecular Biology, 74:277 ― 302
Harmsen and De Haard, Appl Microbiol Biotechnol. 77(1):13-22 (2007)
Van der Linden et al., J Immunol Methods., 240(1-2):185-95 (2000)
Tanha et al., J Immunol Methods. 2002 May 1;263(1-2):97-109
Yau et al., J Immunol Methods. 2003 Oct 1;281(1-2):161-75
Verheesen et al., Biochim Biophys Acta. 2006 Aug;1764(8):1307-19
Goldman et al., Anal Chem. 2006 Dec 15;78(24):8245-55
Greenberg et al., Nature, 347(6518):168-173 (1995)
Shao et al., Mol Immunol. 2007 Jan;44(4):656-65
Zimmer, 2002, Chem. Rev. 102: 759-781
Zhang et al., 2002, Nature Reviews 3: 906-918
Tsien, 1998, Annual Review of Biochemistry 67: 509-544
Tsien et al., U.S. Patent Appn. 200201231 13A1
Thastrup et al., U.S. Patent Appn. 20020107362A1
Bjorn et al., U.S. Patent Appn. 20020177189A1
Fisher, U.S. Patent No. 6,414,119
Ward et al., U.S. Patent Appn. 20030013849
Matz et al., 1999, Nat. Biotechnol. 17:969-973
Wiehler et al., 2001, FEBS Letters 487: 384-389
Terskikh et al., 2000, Science 290: 1585-1588
Baird et al., 2000, Proc. Natl. Acad. Sci. USA 97: 11984-11989
Ando et al., 2002, Proc. Natl. Acad. Sci. USA 99: 12651 -12656
Wiedenmann et al., 2000, Proc. Natl. Acad. Sci. USA 97: 14091-14096
Hongbin et al., 2003, Biochem. Biophys. Res. Commun. 301: 879-885
Lukyanov et al., 2000, J. Biol. Chem. 275: 25879-25882
Dove and Hoegh-Guldberg, 1999, PCT application WO 00146233
Dove et al., 2001, Coral Reefs 19: 197-204
Beddoe et al., 2003, Acta Cryst. D59: 597-599
Bulina et al., 2002, BMC Biochem. 3: 7
Gurskaya et al., 2001, FEBS Letters 507: 16-20

## Claims

1. A method for the detection of the presence or absence of a target in a cell, the method comprising the steps of:
(a) introducing into a cell
(i) a first molecule comprising or consisting of a first single-domain antibody binding to a first portion of the target to be detected and a first detection moiety or a nucleic acid encoding said first molecule in expressible form; and
(ii) a second molecule comprising or consisting of a second single-domain antibody binding to a second portion of said target to be detected and a second detection moiety or a nucleic acid encoding said second molecule in expressible form,
wherein said first portion and said second portion are spatially distinct to allow simultaneous binding of said first and said second single-domain antibody to said target; and wherein said first and said second detection moiety are in combination suitable for generating a detectable fluorescent signal occurring as a consequence of fluorescent moiety Förster resonance energy transfer (FRET) or bimolecular fluorescence complementation (BiFC) when in spatial proximity of each other upon binding of said first molecule to said first portion and said second molecule to said second portion of the target to be detected;
(b) subjecting the cell of step (a) to conditions suitable for generating said detectable fluorescent signal; and
(c) analysing the cell of step (b) for the presence of said detectable fluorescent signal generated by way of fluorescent moiety Förster resonance energy transfer (FRET) or bimolecular fluorescence complementation (BiFC) occurring between said first and said second detection moiety, and/or for a decrease in fluorescence lifetime of the detection moiety acting as energy donor as a consequence of Förster resonance energy transfer (FRET) occurring between said first and said second moiety in comparison to a control, wherein if said detectable fluorescent signal and/or said decrease in fluorescence lifetime can be detected, it is indicative for the presence of said target to be detected in said cell, and wherein if said detectable fluorescent signal and/or decrease in fluorescence lifetime cannot be detected in said cell, it is indicative for the absence of said target to be detected in said cell.

2. The method of claim 1, further comprising the step of quantifying the target detected.

3. The method of any one of claims 1 or 2, wherein said first and second single-domain antibody are each independently selected from a V_{H}H fragment derived from a camelid antibody or a V_{NAR} fragment from a chondrichthyes antibody.

4. The method of any one of claims 1 to 3, wherein said first and second detection moieties are independently from each other selected from the group consisting of fluorescent proteins, quantum dots, and non-protein organic fluorophores, if FRET is to occur; or wherein said first and second fluorescent moieties are complementing fragments of fluorescent proteins selected from the group consisting of YFP, CFP, GFP and Venus.

5. The method of any one of claims 1 to 4, wherein the target comprises a first and a second binding partner and the first portion bound by the first single-domain antibody is present only on the first binding partner and the second portion bound by the second single-domain antibody is present only on the second binding partner, and wherein said detectable fluorescent signal is generated in step (b) of claim 1 upon binding of the first binding partner to the second binding partner.

6. The method of any one of claims 1 to 4, wherein the target comprises a first and a second binding partner and the first portion bound by the first single-domain antibody is present on the first binding partner and the second portion bound by the first single-domain antibody is present on the second binding partner, wherein said first and second portion are the same, and wherein said detectable fluorescent signal is generated in step (b) of claim 1 upon binding of the first binding partner to the second binding partner.

7. The method of any one of claims 1 to 4, wherein said first and second binding portions are located on the target to be detected in a manner so that said detectable fluorescent signal is generated in step (b) of claim 1 upon a conformational change of the target.

8. A composition comprising a first molecule and a second molecule or one or more nucleic acids encoding said first or second molecule according to any one of claims 1 to 7.

9. A kit comprising a first molecule and a second molecule or one or more nucleic acids encoding said first or second molecule according to any one of claims 1 to 7 or the composition according to claim 8 and, optionally, instructions for use.
